Europäisches Patentamt

European Patent Office

Office européen des brevets

(19)

(11) Veröffentlichungsnummer : **0 118 832**
**B1**

(12) EUROPÄISCHE PATENTSCHRIFT

(45) Veröffentlichungstag der Patentschrift :
02.01.86

(21) Anmeldenummer : 84102120.7

(22) Anmeldetag : 29.02.84

(51) Int. Cl.⁴ : **C 07 C143/30**

(54) Verfahren zur Herstellung des Dinatriumsalzes der Naphthalin-1,5-disulfonsäure.

(30) Priorität : **12.03.83 DE 3308883**

(43) Veröffentlichungstag der Anmeldung :
**19.09.84 Patentblatt 84/38**

(45) Bekanntmachung des Hinweises auf die Patenterteilung : **02.01.86 Patentblatt 86/01**

(84) Benannte Vertragsstaaten :
**CH DE FR GB LI**

(56) Entgegenhaltungen :
**EP-A- 0 012 260**
**"Ullmanns Encyklopädie der technischen Chemie", 4.**
**Auflage, Band 17, "Milchsäure bis Petrolkoks", Seite**
**118, Verlag Chemie, Weinheim, DE.**

(73) Patentinhaber : **BAYER AG**
**Konzernverwaltung RP Patentabteilung**
**D-5090 Leverkusen 1 Bayerwerk (DE)**

(72) Erfinder : **Behre, Horst, Dr.**
**Zur alten Linde 12**
**D-5068 Odenthal-Eikamp (DE)**
Erfinder : **Blank, Heinz Ulrich, Dr.**
**Am Geusfelde 35**
**D-5068 Odenthal (DE)**
Erfinder : **Hammerschmidt, Erich, Dr.**
**Schützenstrasse 25**
**D-5060 Bergisch Gladbach 2 (DE)**

EP 0 118 832 B1

## Beschreibung

Die Erfindung betrifft ein neues Verfahren zur Herstellung des Dinatriumsalzes der Naphthalin-1.5-disulfonsäure.

Das Dinatriumsalz der Naphthalin-1.5-disulfonsäure ist ein wichtiges Zwischenprodukt zur Herstellung von Farbstoffen und Polyurethanen (s. z. B. Ullmanns Enzyklopädie der technischen Chemie, 4. Auflage, Band 17, S. 108 und 118).

Das Dinatriumsalz der Naphthalin-1.5-disulfonsäure ist z. B. durch Umsetzung der freien Naphthalin-1.5-disulfonsäure mit Soda erhältlich (s. Helv. Chimica Acta, Vol. VI, (1923), S. 1137-1138). Hergestellt wurde das Dinatriumsalz der Naphthalin-1.5-disulfonsäure bislang durch Aussalzen mit Natriumsulfat aus dem bei der Herstellung von Naphthalin-1.5-disulfonsäure anfallenden verdünnten Sulfonierungsgemisch (s. z. B. Ullmanns Enzyklopädie der technischen Chemie, 3. Auflage, Band 12, S. 595). Nachteilig an dieser Herstellung ist, daß man als Abwasser eine salzhaltige wäßrige Schwefelsäure erhält, die eine Aufarbeitung der Schwefelsäure nicht zuläßt.

Es wurde nun gefunden, daß man das Dinatriumsalz der Naphthalin-1.5-disulfonsäure nicht nur durch Aussalzen mit Natriumsulfat aus verdünnten Sulfonierungsgemischen herstellen, d. h. aus salzhaltiger verdünnter Schwefelsäure gewinnen kann, sondern auch durch Neutralisation bestimmter Sulfonierungsgemische, d. h. aus neutralen wäßrigen Natriumsulfatlösungen und dies nicht nur in hohen Ausbeuten sondern auch in hoher Reinheit. Überraschenderweise wurde festgestellt, daß bei diesem Ausfällen durch Neutralisation, d. h. aus neutralen wäßrigen Lösungen, die ebenfalls in der Lösung befindlichen Natriumsalze der isomeren Naphthalin-disulfonsäuren und der Naphthalin-mono- und trisulfonsäuren nicht mit ausgefällt werden. Dies ist deshalb überraschend, weil verdünnte Schwefelsäure bekannterweise ein gutes Lösungsmittel für diese als Nebenprodukte anfallenden Sulfonsäuren bzw. deren Natriumsalze darstellt. Es war deshalb zu erwarten, daß beim Ausfällen aus neutralen wäßrigen Lösungen, d. h. Abwesenheit des Lösungsmittels Schwefelsäure, nicht nur das Dinatriumsalz der Naphthalin-1.5-disulfonsäure sondern auch die Natriumsalze der anderen Sulfonsäuren mit ausgefällt würden.

Die Erfindung betrifft daher ein Verfahren zur Herstellung des Dinatriumsalzes der Naphthalin-1.5-disulfonsäure aus bei der Herstellung von Naphthalin-1.5-disulfonsäure anfallenden, gegebenenfalls verdünnten, Sulfonierungsgemischen, das dadurch gekennzeichnet ist, daß man als Sulfonierungsgemische solche einsetzt, wie sie bei der Sulfonierung von Naphthalin mit 2,3-5,0 Mol Schwefeltrioxid je Mol Naphthalin in einem inerten organischen Lösungsmittel erhalten werden und daß man diese Sulfonierungsgemische mit Natriumhydroxid oder -carbonat auf einen pH-Wert von 5 bis 9, vorzugsweise 6 bis 8, neutralisiert und das sich abscheidende Dinatriumsalz in an sich bekannter Weise aus der bei der Neutralisation entstandenen Suspension isoliert.

Das erfindungsgemäße Verfahren eignet sich besonders für die Herstellung des Dinatriumsalzes aus den bei der Herstellung von Naphthalin-1.5-disulfonsäure anfallenden Sulfonierungsgemischen, die bei der Umsetzung von Naphthalin mit nur 2,8 bis 3,6 Mol $SO_3$ je Mol Naphthalin, in einem inerten organischen Lösungsmittel erhalten werden und beispielsweise in der EP-A-0 012 260 beschrieben sind.

Diese bevorzugt einzusetzenden Sulfonierungsgemische enthalten Naphthalinsulfonsäuren und Schwefelsäure in einem molaren Verhältnis von 1 : 1 bis 1 : 3, bevorzugt 1 : 1,2 bis 1 : 1,6. Die Sulfonierungsgemische sind vorzugsweise mit Wasser verdünnt und enthalten 20 bis 80 Gew.-%, vorzugsweise 35 bis 75 Gew.-%, Wasser, bezogen auf das Gewicht des Gesamtgemisches.

Die erfindungsgemäß vorzugsweise zu verwendenden Sulfonierungsgemische enthalten neben der Naphthalin-1.5-disulfonsäure noch isomere Naphthalinsulfonsäuren, z. B. Naphthalin-1.3-, -1,6- und -1,7-disulfonsäure, ferner Naphthalintrisulfonsäuren, z. B. Naphthalin-1.3.5-, -1.3.6- und -1.3.7-trisulfonsäure und gegebenenfalls auch noch Naphthalin-1-sulfonsäure.

Der Anteil der einzelnen Sulfonsäuren, ausgenommen der Anteil der Naphthalin-1.5-disulfonsäure, darf 40 Mol-% der insgesamt im Sulfonierungsgemisch enthaltenen Sulfonsäuren nicht überschreiten.

Die in dem erfindungsgemäßen Verfahren einzusetzenden Sulfonierungsgemische können sowohl in Form von Lösungen als auch in Form von Suspensionen vorliegen. Für die Neutralisation können entweder die Base oder aber das wäßrige Sulfonierungsgemisch vorgelegt und die jeweils andere Komponente zugesetzt werden. Ferner kann man Sulfonierungsgemisch und Base gleichzeitig in eine Vorlage einbringen (simultane Neutralisation). Die erfindungsgemäße Neutralisation wird bei Temperaturen von 30 bis 110 °C, vorzugsweise von 50 bis 100 °C, ausgeführt.

Natriumhydroxid oder Natriumcarbonat können gegebenenfalls in Form ihrer wäßrigen Lösungen eingesetzt werden.

Die Isolierung des Dinatriumsalzes der Naphthalin-1.5-disulfonsäure wird vorteilhaft bei Temperaturen von 0 bis 50 °C, vorzugsweise von 20 bis 40 °C, vorgenommen.

Mit Hilfe des erfindungsgemäßen Verfahrens wird unmittelbar, d. h. ohne weitere Reinigungsoperationen, ein spezifikationsgerechtes Dinatriumsalz der Naphthalin-1.5-disulfonsäure erhalten. Gegenüber der bekannten Gewinnung des Dinatriumsalzes der Naphthalin-1.5-disulfonsäure durch Aussalzen aus verdünnten Sulfonierungsgemischen bietet das erfindungsgemäße Verfahren entscheidende Vorteile : Es entfällt das Neutralwaschen des abgetrennten Dinatriumsalzes mit Sodalösung. Es wird die Bildung des schlecht absaugbaren Naphthalin-1.5-disulfonsäuremononatriumsalzes vermieden. Das Natriumsulfat läßt sich aus dem Ablauf zurückgewinnen.

Gegenüber der Herstellung des Dinatriumsalzes der Naphthalin-1.5-disulfonsäure durch Neutralisation des Naphthalin-1.5-disulfonsäuretetrahydrates bietet das erfindungsgemäße Verfahren den überraschenden Vorteil, daß nach ihm ein Dinatriumsalz erhalten wird, dessen Natriumsulfatgehalt unter 1 Gew.-%, bezogen auf das Gewicht des trockenen Salzes, liegt. D. h. bei der Neutralisation der reinen Naphthalin-1.5-disulfonsäure wird ein weniger reines Produkt erhalten als bei der Neutralisation des die Naphthalin-1.5-disulfonsäure enthaltenden Sulfonierungsgemisches. Der geringe Natriumsulfat-Gehalt ist für die Weiterverarbeitung des Salzes zu 1.5-Dihydroxynaphthalin von entscheidender Bedeutung.

Beispiel 1

758 g wässriges Naphthalin-1.5-disulfonsäure-sulfonierungsgemisch (erhalten durch Sulfonierung von 1 Mol Naphthalin mit 3,2 Molen Schwefeltrioxid in Methylenchlorid unter den in Beispiel 1 der EP-A-0 012 260 beschriebenen Bedingungen), Verdünnen des Sulfonierungsgemisches mit Wasser und Abtrennen des Methylenchlorids ; Zusammensetzung des Gemisches : 29,5 Gew.-% Naphthalin-1.5-disulfonsäure, 5,2 Gew.-% Naphthalin-1.6-disulfonsäure, 1,8 Gew.-% Naphthalin-1.7-disulfonsäure, 0,8 Gew.-% Naphthalin-1.3.5-trisulfonsäure, 0,5 Gew.-% Naphthalin-1.3.6-trisulfonsäure, 19 Gew.-% Schwefelsäure, 43 Gew.-% Wasser ; molares Verhältnis Naphthalinsulfonsäuren : Schwefelsäure = 1 : 1,5) werden vorgelegt und unter Rühren bei 60 °C mit 50 %iger Natronlauge bis zu einem pH-Wert von 7,5 neutralisiert.

Die Mischung wird unter Rühren auf 25 °C abgekühlt. Anschließend wird das Dinatriumsalz abgesaugt und mit 200 ml 10 %iger Natriumsulfatlösung gewaschen. Ausbeute : 94 % der Theorie, bezogen auf die Menge der im Sulfonierungsgemisch enthaltenen Naphthalin-1.5-disulfonsäure. Das getrocknete Produkt ist 95,5 %ig (Gehalt an Verunreinigungen, 0,4 % Trinatriumsalz der Naphthalin-1.3.5-trisulfonsäure ; 0,74 % $Na_2SO_4$ ; 3,0 % Wasser).

Beispiel 2

1 388 g eines wäßrigen Naphthalin-1,5-disulfonsäure-sulfonierungsgemisches (erhalten durch Sulfonierung von 1 Mol Naphthalin mit 4,8 Mol Schwefeltrioxid in Methylenchlorid, Verdünnen des Sulfonierungsgemisches mit Wasser und Abtrennen des Methylenchlorids ; Zusammensetzung des Gemisches : 15,9 Gew.-% Naphthalin-1,5-disulfonsäure, 2,8 Gew.-% Naphthalin-1,6-disulfonsäure, 1,0 Gew.-% Naphthalin-1,7-disulfonsäure, 0,8 Gew.-% Naphthalin-1,3,5-trisulfonsäure, 0,5 Gew.-% Naphthalin-1,3,6-trisulfonsäure, 19,5 Gew.-% Schwefelsäure, 59,4 Gew.-% Wasser ; molares Verhältnis Naphthalinsulfonsäuren : Schwefelsäure = 1 : 2,8) werden vorgelegt und unter Rühren bei 75 °C mit 605 g 50 %-iger NaOH bis zu einem pH-Wert 7 neutralisiert.

Die Mischung wird unter Rühren auf 30 °C abgekühlt und das Dinatriumsalz der Armstrongsäure abgesaugt. Nach dem Waschen mit 200 ml 10 %-iger Natriumsulfatlösung beträgt die Ausbeute 90,5 % d. Th., bezogen auf die Menge der im Sulfonierungsgemisch enthaltenen Naphthalin-1,5-disulfonsäure. Das getrocknete Produkt ist 95,7 %-ig (Gehalt an Verunreinigungen : 0,3 % Trinatriumsalz der Naphthalin-1,3,5-trisulfonsäure ; 0,96 % $Na_2SO_4$ ; 2,8 % Wasser).

**Patentansprüche**

1. Verfahren zur Herstellung des Dinatriumsalzes der Naphthalin-1.5-disulfonsäure aus bei der Herstellung von Naphthalin-1,5-disulfonsäure anfallenden, gegebenenfalls mit Wasser verdünnten Sulfonierungsgemischen, dadurch gekennzeichnet, daß man als Sulfonierungsgemische solche einsetzt, wie sie bei der Sulfonierung von Naphthalin mit 2,3-5,0 Mol Schwefeltrioxid je Mol Naphthalin in einem inerten organischen Lösungsmittel erhalten werden, und daß man diese Sulfonierungsgemische mit Natriumhydroxid oder Natriumcarbonat auf einen pH-Wert von 5 bis 9 neutralisiert und das sich abscheidende Dinatriumsalz in an sich bekannter Weise aus der bei der Neutralisation entstehenden Suspension isoliert.

2. Verfahren gemäß Anspruch 1, dadurch gekennzeichnet, daß man als Sulfonierungsgemisch ein solches einsetzt, wie es bei der Sulfonierung von Naphthalin mit 2,8-3,6 Mol Schwefeltrioxid je Mol Naphthalin in einem inerten organischen Lösungsmittel erhalten wird.

3. Verfahren gemäß Anspruch 1, dadurch gekennzeichnet, daß das zu neutralisierende Sulfonierungsgemisch Naphthalinsulfonsäuren und Schwefelsäure im molaren Verhältnis von 1 : 1 bis 1 : 3 enthält.

4. Verfahren nach Ansprüchen 1 bis 3, dadurch gekennzeichnet, daß das zu neutralisierende Sulfonierungsgemisch 20 bis 80 Gew.-% Wasser, bezogen auf das Gesamtgemisch, enthält.

**Claims**

1. Process for the preparation of the disodium salt of naphthalene-1,5-disulphonic acid from sulphonation mixtures which are obtained in the preparation of naphthalene-1,5-disulphonic acid and are possibly diluted with water, characterised in that the sulphonation mixtures used are those such as are obtained in the sulphonation of naphthalene with 2.3-5.0 moles of sulphur trioxide per mole of naphthalene in an inert organic solvent, and in that these sulphonation mixtures are neutralised with sodium hydroxide or sodium carbonate to a pH value of 5 to 9 and the disodium salt which precipitates is isolated, in a manner known per se, from the suspension formed during the neutralisation.

2. Process according to Claim 1, characterised in that the sulphonation mixture used is one such as is obtained in the sulphonation of naphthalene with 2.8-3.6 moles of sulphur trioxide per mole of naphthalene in an inert organic solvent.

3. Process according to Claim 1, characterised in that the sulphonation mixture to be neutralised contains naphthalene sulphonic acids and sulphuric acid in a molar ratio of 1 : 1 to 1 : 3.

4. Process according to Claims 1 to 3, characterised in that the sulphonation mixture to be neutralised contains 20 to 80 % by weight of water, based on the total mixture.

## Revendications

1. Procédé de préparation du sel disodique de l'acide naphtalène-1,5-disulfonique à partir de mélanges de sulfonation obtenus à la préparation de l'acide naphtalène-1,5-disulfonique, éventuellement dilués par l'eau, caractérisé en ce que l'on met en œuvre en tant que mélanges de sulfonation les mélanges de sulfonation obtenus à la sulfonation du naphtalène par 2,3 à 5,0 moles d'anhydride sulfurique par mole de naphtalène dans un solvant organique inerte, en ce que l'on neutralise ces mélanges de sulfonation par l'hydroxyde de sodium ou le carbonate de sodium jusqu'à un pH de 5 à 9 et en ce que l'on isole de la suspension obtenue à la neutralisation, de manière connue en soi, le sel disodique qui s'est séparé.

2. Procédé selon la revendication 1, caractérisé en ce que le mélange de sulfonation mis en œuvre a été obtenu à la sulfonation du naphtalène par 2,8 à 3,6 moles d'anhydride sulfurique par mole de naphtalène dans un solvant organique inerte.

3. Procédé selon la revendication 1, caractérisé en ce que le mélange de sulfonation à neutraliser contient des acides naphtalène-sulfoniques et de l'acide sulfurique dans un rapport molaire de 1 : 1 à 1 : 3.

4. Procédé selon les revendications 1 à 3, caractérisé en ce que le mélange de sulfonation à neutraliser contient 20 à 80 % en poids d'eau, par rapport au mélange total.